# EUROPEAN PATENT APPLICATION

(11) **EP 2 070 521 A1**
(43) Date of publication of application: **17.06.2009**
(21) Application number: 07076065.7
(22) Date of filing: 10.12.2007
(51) Int. Cl.: A61K 9/51, A61K 31/427

(54) **Surface-modified nanoparticles**

(71) Applicant: Bayer Schering Pharma Aktiengesellschaft, 13353 Berlin (DE)
(72) Inventor: General, Sascha, Dr., 10178 Berlin (DE)
(74) Representative: Krüger, Anita

(57) **Abstract**

The present invention relates to compositions of drug-bearing nanoparticles having a modified surface, pharmaceutical kits containing the individual components of said compositions, the medical uses thereof, as well as a method for producing said compositions.

## Description

### Field of the invention

The present invention relates to compositions of drug-bearing nanoparticles having a modified surface, pharmaceutical kits containing the individual components of said compositions, the medical uses thereof, as well as a method for producing said compositions.

### Background of the invention

Many potential drugs are never marketed because of low bioavailability. Drug developers are often faced with the challenge of having to deal with drug candidates of poor stability, low solubility, low permeability through biomembranes etc.

Incorporation of the drug load into surface-modified nanoparticles has been proposed as one way of overcoming the problems associated with low solubility and low permeability through biomembranes.

Published PCT application WO 95/22963 discloses nanoparticles coated with a surfactant for delivering drugs across the blood-brain barrier or blood-nerve barrier. WO 03/094894 discloses nanoparticulate compositions comprising nystatin, an antibiotic, with a surface stabiliser adsorbed to the surface of the nanoparticle. US 6,288,040 B1 discloses particles that are modified by binding a recognition protein to the particle surface, thereby improving the delivery of drugs to the central nervous system (CNS). WO 2005/110407 discloses compositions of nanoparticles containing epothilone-like lactam compounds, said nanoparticles having a stabilizer adsorbed on the surface of the particles. The stabilizers aid in the wetting or stabilization of the nanoparticles.

So far, most of the attention in the field has been given to the relationship between the surface-modifying substance adsorbed to the nanoparticles and the surrounding environment. The present invention also addresses the problems associated with insufficient binding of the surface-modifying substance to the surface of the nanoparticles.

By addressing this problem, the present invention addresses the problem of achieving homogeneous aqueous solutions or suspensions of active drug ingredients that are poorly soluble in water. Also, addressing the issue of adherence of the surface-modifying substance to the surface of the nanoparticle, means that the dimensions of the modified nanoparticles may be adjusted in such a way that the skilled person would expect the modified particles to have a long circulation time in the blood stream. Said adjustment of the particle size would also lead the skilled person to expect accumulation of the active drug ingredient in e.g. tumor tissue, allowing passive targeting of tumor cells.

The surface-modified nanoparticles of the present invention solve these problems by providing nanoparticles modified by binding a modifying substance thereto, said substance being selected based on the properties of the non-modified nanoparticle relative to the properties of the substance.

### Summary of the invention

The present invention relates in one aspect to a pharmaceutical composition comprising surface-modified nanoparticles, wherein the core of said nanoparticles is a non-modified nanoparticle containing an active drug substance, and wherein one or more positively charged block-copolymers are bound to the surface of said nanoparticles

In another aspect, the present invention concerns a pharmaceutical kit comprising:
i) A first composition comprising nanoparticles, wherein said nanoparticles contain an active drug substance; and
ii) a second composition comprising a positively charged block-copolymer. In a further aspect the invention concerns a pharmaceutical kit comprising a composition containing surface-modified nanoparticles.

In yet another aspect of the invention said composition comprises surface modified particles which are in solid form, e.g. lyophilized and the related pharmaceutical kit comprises the solid surface-modified nanoparticles together with means for reconstitution.

In another aspect of the invention said composition comprises
(a) one portion of non-modified particles which are optionally in solid form, e.g. lyophilized, together with
(b) a second portion comprising a block co-polymer optionally diluted with water for injection or infusion or an aqueous salt containing solution e.g. physiological saline solution and optionally
(c) means for reconstitution of optionally present solid forms.

Means for reconstitution may be e.g. water suitable for injection or infusion, physiological saline solution.

In yet another aspect, the present invention relates to a compositions according to the previous aspect for use as a medicament.

In still another aspect, the present invention concerns use of a composition according to the previous aspect for the manufacture of a medicament for the treatment of cancer.

In an alternative aspect, the present invention concerns a method for the treatment of cancer, said method comprising administering a composition according to the previous aspect to a patient in need thereof.

In yet another aspect, the present invention defines a method for the preparation of the composition according to nanoparticles of the invention comprising the steps
a) Dissolving an active drug substance in an organic solvent;
b) Dissolving a biodegradable polymer or a mixture of biodegradable polymers in an organic solvent;
c) Combining solutions a) and b);
d) Diluting c) with or adding c) to a non-solvent of the drug and the biodegradable polymer(s) to form the non-modified nanoparticles
e) Optionally lyophilizing d) in the presence of a cryoprotector or lyoprotector;
f) Optionally diluting d) or e) with water (reconstitution);
g) Optionally removing the organic solvent of d) or f) using e.g. temperature methods (evaporation) or filtration methods (e.g. microdialysis) and optionally adding water to adjust the volume of the dispersion
h) Optionally removing unencapsulated epothilone by suitable methods e.g. filtration by using ultracentrifugation methods
i) Optionally adding cryoprotector or lyoprotector; lyophilisation of g) or h)
j) Preparing an aqueous solution of a positively charged block-copolymer; and
k) Combining d)?, f) and j) or g) and j) in any order possible optionally adding water thereto or adding both into water.
l) Optionally lyophilizing k)
m) Optionally reconstitution with water for injection or infusion or with physiological saline solution.

One aspect of the invention is a process having the following steps: a), b), c), d), g) (stirring at suitable temperature), j), k) combining g) and j) as disclosed in example 1.

Another aspect of the invention is the process following the steps:
a') Dissolving active drug substance and biodegradable polymer or a mixture of biodegradable polymers together in an organic solvent
d) whereby the non-solvent may comprise a surfactant (such as e.g. lecitine, synperonics such as e.g. synperonic F68 or Tween such as e.g. Tween 60, Tween 80)as an additive,
g), h),
adding h) to water,
i), redispersion in water for injection/sodiumchloride or water for injection adding j) to the dispersion obtained,
I), m) as disclosed in example 2.

### Definitions

In the context of the present invention, the term "surface-modified nanoparticle" is meant to include nanoparticles commonly known in the art, wherein one or more polymers are bound to the surface of said nanoparticle. The core of the surface modified nanoparticle is also called a non-modified nanoparticle.

In the context of the present invention, the term "active drug substance" is used in the usual meaning in the art. In particular it refers to anti-tumor compounds, especially epothilones or epothilone analogues, e.g. epothilones or epothilone analogues as disclosed in the publications as cited below as well as epothilone A, epothilone B (= Epo-906 = Patupilone), epothilone C, epothilone D (= Kos-862), epothilone E, epothilone F, (E)-9,10-dehydro epothilone B (= KOS-1584), KOS-1803, 15-aza-epothilone B (= BMS-247550 = Ixabepilone), BMS-310705, ABJ 879, fludelone.

The term "epothilones or epothilone analogues" refers to a class of compounds, the bounds of which are known to the person skilled in the art e.g. from WO 93/10102, WO 93/10121 and DE 41 38 042 A2, WO 97/19086 and WO 98/25929 , WO 99/43320, WO 2000/066589, WO 00/49021 WO 00/71521 , WO 2001027308 , WO 99/02514 , WO 2002080846.

The epothilones and their derivatives as being suitable for the present invention as well as the production thereof is disclosed, for example in DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly interesting epothilones of the present invention may be produced according to WO 00/66589.

Preferred compounds covered by the term "epothilones or epothilone analogues" may in particular be defined as a compound of the formula: 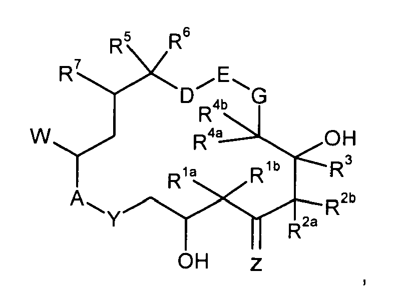 wherein
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or together form a -(CH₂)ₚ- group where p is 2 to 5;
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal, CHal₃; Hal is a halogen atom
- R⁶,: R⁷ are each hydrogen, or together form an additional bond, or together form an epoxy function;
- G: is O or CH₂;
- D-E: together is a group -H₂C-CH₂-, -HC=CH-, -C≡C-, -CH(OH)-CH(OH)-, -CH(OH)-CH₂-, -CH₂-CH(OH)-, -CH₂-O-, -O-CH₂- or 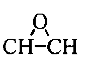 whereby if G is O then D-E cannot be CH₂-O; or
D-E-Gtogether is a group H₂C-CH=CH
- W: is a group C(=X)R⁸, or is a bi- or tricyclic aromatic or heteroaromatic radical; X is O or a group CR⁹R¹⁰ and
- R⁸: is hydrogen, C₁-C₁₀ alkyl, aryl, aralkyl, halogen, CN;
- R⁹, R¹⁰: are each independently hydrogen, C₁-C₂₀ alkyl, aryl, aralkyl, or together with the methylene carbon form a 5- to 7-membered carbocyclic or heterocyclic aromatic or non-aromatic ring;
- Z: is O or H and the group OR¹¹;
- R¹¹: is hydrogen or a protecting group PG^{z};
- A-Y: is a group O-C(=O), O-CH₂, CH₂-C(=O), NR¹²-C(=O), NR¹²-SO₂;
- R¹²: is hydrogen, or C₁-C₁₀ alkyl;
- PG^{z}: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers,and / or as a pharmaceutically acceptable salt thereof.

Another embodiment for the nanoparticles of the invention are nanoparticles wherein the encapsulated compounds are epothilones of formula (I) wherein
- R^{1a}, R^{1b}: are each independently hydrogen, C₁-C₄ alkyl, or together form a -(CH₂)ₘ-group where m is 2 to 5;
- R^{2a}, R^{2b}: are each independently hydrogen, C₁-C₅ alkyl, or together form a -(CH₂)ₙ-group where n is 2 to 5, or C₂-C₆ alkenyl, or C₂-C₆ alkynyl;
- R³: is hydrogen,
- R^{4a}, R^{4b}: are each independently hydrogen, C₁-C₄ alkyl,
- R⁵: is hydrogen, C₁-C₄ alkyl, C(Hal)₃
- R⁶, R⁷: are each hydrogen, or together form an additional bond, or together form an epoxy function;
- G: is CH₂;
- D-E: is a group H₂C-CH₂, or
- D-E-G: together is a group H₂C-CH=CH
- W: is a group C(=X)R⁸, or is a bi- or tricyclic aromatic or heteroaromatic radical;
- X: is a group CR⁹R¹⁰;
- R⁸: is hydrogen, C₁-C₄ alkyl, halogen,
- R⁹, R¹⁰: are each independently hydrogen, C₁-C₄ alkyl, aryl, aralkyl,
- Z: is O
- A-Y: is a group O-C(=O), NR¹²-C(=O)
- R¹²: is hydrogen, or C₁-C₄ alkyl;
as a single stereoisomer or a mixture of different stereoisomers, and / or as a pharmaceutically acceptable salt thereof.

Still another aspect of the invention are nanoparticles comprising epothilones of formula (I),
wherein
- R^{1a}, R^{1b}: are each independently hydrogen, C₁-C₂ alkyl, or together form a -(CH₂)ₘ-group where m is 2 to 5,
- R^{2a}, R^{2b}: are each independently hydrogen, C₁-C₅ alkyl, or together form a -(CH₂)ₙ-group where n is 2 to 5, or C₂-C₆ alkenyl, or C₂-C₆ alkynyl;
- R³: is hydrogen,
- R^{4a}, R^{4b}: are each independently hydrogen, C₁-C₂ alkyl,
- R⁵: is hydrogen or methyl or trifluormethyl,
- R⁶, R⁷: together form an additional bond, or together form an epoxy function;
- G: is CH₂;
- D-E: is a group H₂C-CH₂, or
- D-E-G: together is a group H₂C-CH=CH
- W: is a group C(=X)R⁸, or is thiazolyl, oxazolyl, pyridyl, N-oxo-pyridyl; benzothiazolyl, benzoxazolyl, benzimidazolyl, which are optionally substituted by C₁-C₃-alkyl, C₁-C₃-hydroxyalkyl, C₁-C₃-aminoalkyl, C₁-C₃-alkylsulfonyl
- X: is a group CR⁹R¹⁰;
- R⁸: is hydrogen, methyl, chloro, fluoro,
- R⁹, R¹⁰: are each independently hydrogen, C₁-C₄ alkyl, thiazolyl, oxazolyl, pyridyl, N-oxo-pyridyl, which are optionally substituted by C₁-C₃-alkyl, C₁-C₃-hydroxyalkyl, aralkyl,
- Z: is O
- A-Y: is a group O-C(=O), NR¹²-C(=O)
- R¹²: is hydrogen, or C₁-C₄ alkyl;
as a single stereoisomer or a mixture of different stereoisomers, and / or as a pharmaceutically acceptable salt thereof.

An embodiment of the invention are nanoparticles comprising compounds of general formula (I), wherein A-Y is O-C(=O); D-E is H₂C-CH₂; G is CH₂; Z is O; R^{1a}, R1^{b} are both C₁-C₁₀ alkyl or form together a -(CH₂)ₚ- group where p is 2 to 3; R^{2a}, R^{2b} are each independently hydrogen, C₁-C₁₀ alkyl, C₂-C₁₀ alkenyl, or C₂-C₁₀ alkynyl; R³ is hydrogen; R^{4a}, R^{4b} are each independently hydrogen or C₁-C₁₀ alkyl; R⁵ is C₁-C₁₀ alkyl.

Another embodiment of the invention are nanoparticles comprising compounds of the general formula (I), wherein R^{2a}, R^{2b} are each independently hydrogen, C₂-C₁₀ alkenyl or C₂-C₁₀ alkynyl; R⁶, R⁷ form an epoxy function or together form an additional bond.

One embodiment of the invention are nanoparticles comprising compounds of formula I wherein W is C(=X)R8 and X is CR⁹R¹⁰ or W is a bi- or tricyclic aromatic radical.

A further embodiment of the invention are nanoparticles comprising compounds of formula I wherein W is a bi- or tricyclic aromatic radical, especially a bicyclic radical.

Still a further aspect of the invention are nanoparticles comprising compounds of formula (I) wherein W is a 2-Methylbenzothiazol-5-yl radical or a 2-Methylbenzoxazol-5-yl radical, most preferred wherein W is a 2-Methylbenzothiazol-5-yl radical.

Another aspect of the invention are nanoparticles comprising compounds of formula (I) wherein R^{2a}, R^{2b} are each independently hydrogen, C₂-C₁₀ alkenyl or C₂-C₁₀ alkynyl; R⁶, R⁷ form an epoxy function and W is a 2-Methylbenzothiazol-5-yl radical or a 2-Methylbenzoxazol-5-yl radical, a pyridyl radical or a Quinoline-7-yl radical.

An aspect of the invention are nanoparticles wherein the encapsulated epothilones are selected from the group consisting of
(4S,7R,8S,9S,13E/Z,16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1 S/R,3S(E),7S, 10R, 11 R, 12S, 16R/S)-7,11-dihydroxy-10-(prop-2-en-1-yl)-3-(2-methyl-benzoxazol-5-yl)-8,8,12,16-tetramethyl-4,17-dioxabicyclo[14.1.0] heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1 S/R,3S,7S,10R,11R, 12S, 16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(4S,7R,8S,9S, 13E/Z, 16S)-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S, 13E/Z, 16S)-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S,7S,10R,11R,12S,16R/S)-7,11-dihydroxy-10-(prop-2-in-1-yl)-3-(2-methyl-benzothiazol-5-yl)-8,8,12,16-tetramethyl-4,17-dioxabicyclo[14.1.0]heptadecane-5,9-dione;
(1S,3S,7S,1 0R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione
(4S,7R,8S,9S, 13E/Z, 16S)-4,8-dihydroxy-16-(chinolin-7-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(chinolin-2-yl)-8,8,12,16-tetramethyl-4,17-dioxabicyclo[14.1.0] heptadecane-5,9-dione;
(1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0] heptadecane-5,9-dione;
(4S,7R,8S,9S,13E/Z,16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; and
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4-aza-17-oxabicyclo[14.1.0]heptadecane-5,9-dione.Another aspect of the invention are nanoparticles wherein the encapsulated epothilones are selected from the group consisting of
(4S,7R,8S,9S, 13E/Z, 16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzoxazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S,7S,10R,11S,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione; (1S,3S,7S, 10R, 11S, 12S, 16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(4S,7R,8S,9S, 13E/Z, 16S)-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S,7S,10R,11R,12S,16R/S)-7,11-dihydroxy-10-(prop-2-in-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(quinolin-7-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(quinolin-7-yl)-8,8,12,16-tetramethyl-4,17-dioxabicyclo[14.1.0]heptadecane-5,9-dione;
(4S,7R,8S,9S, 13E/Z, 16S)-4,8-dihydroxy-16-(1,2-dimethyl-1H-benzoimidazol-5-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(1,2-dimethyl-1 H-benzoimidazol-5-yl)-8,8,12,16-tetramethyl-4,17-dioxabicyclo[14.1.0]heptadecane-5,9-dione;
(4S,7R,8S,9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1S/R,3S,7S,10R,11S,12S,16R/S)-7,11-dihydroxy-10-(prop-2-en-1-yl)-3-(2-methyl-benzothiazol-5-yl)-8,8,12,16-tetramethyl-4-aza-17-oxabicyclo[14.1.0]heptadecane-5,9-dione, and
(1S/R,3S,7S, 10R,11R,12S,16R/S)-7,11-dihydroxy-10-(prop-2-en-1-yl)-3 -2-(2-methyl-benzothiazol-5-yl)-8,8,12,16-tetramethyl-4-aza-17-oxabicyclo[14.1.0]heptadecane-5,9-dione,
(1S,3S(E),7S,10R,11S,12S,16R)-7,11-Dihydroxy-8,8,10,12,16-pentamethyl-3-[1-(2-methyl-1,3-thiazol-4-yl)prop-1-en-2-yl]-4,17-dioxabicyclo[14.1.0]heptadecane-5,9-dione,
(1S,3S(E),7S,10R,11S, 12S,16R)-7,11-Dihydroxy-8,8,10,12,16-pentamethyl-3-[1-(2-methyl-1,3-thiazol-4-yl)prop-1-en-2-yl]-17-oxa-4-azabicyclo[14.1.0]heptadecane-5,9-dione,
(4S,7R,8S,9S,13Z,16S(E))-4,8-dihydroxy-5,5,7,9,13-pentamethyl-16-[1-(2-methyl-1,3-thiazol-4-yl)prop-1-en-2-yl]-oxacyclohexadec-13-ene-2,6-dione,
(4S,7R,8S,9S, 10E,13Z,16S(E))-4,8-dihydroxy-5,5,7,9,13-pentamethyl-16-[1-(2-methyl-1,3-thiazol-4-yl)prop-1-en-2-yl]-oxacyclohexadec-10,13-diene-2,6-dione,
(4S,7R,8S,9S,10E,13Z,16S(E))-4,8-dihydroxy-5,5,7,9-tetramethyl-13-rifluormethyl-16-[1-(2-methyl-1,3-thiazol-4-yl)prop-1-en-2-yl]-oxacyclohexadec-10,13-diene-2,6-dione,
(1S,3S(E),7S,1 0R,11S,12S,16R)-7,11-Dihydroxy-8,8,10,12,16-pentamethyl-3-[1-(2-methylsulfanyl-1,3-thiazol-4-yl)prop-1-en-2-yl]-4,17-dioxabicyclo[14.1.0]heptadecane-5,9-dione
as a single stereoisomer or a mixture of different stereoisomers, and / or as a pharmaceutically acceptable salt thereof.

One aspect of the invention are nanoparticles wherein the encapsulated epothilones are selected from the group consisting of (4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione; (1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(1S/R, 3S,7S, 10R,11S,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione
(1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione or any stereoisomer thereof as a single stereoisomer or a mixture of different stereoisomers, and / or as a pharmaceutically acceptable salt thereof..

Another preferred embodiment of the invention are nanoparticles wherein the encapsulated epothilone is (1S,3S,7S,10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione .or any stereoisomer or diastereoisomer thereof as a single stereoisomer or a mixture of different stereoisomers, and / or as a pharmaceutically acceptable salt thereof..

The term "alkyl" as used herein refers to straight-chain or branched-chain alkyl groups with 1-20 carbon atoms, e. g., methyl, ethyl, propyl, isopropyl, n-butyl, t-butyl, n-pentyl, isopentyl, neopentyl, heptyl, hexyl and decyl. Alkyl groups can be perfluorated or substituted by one to five substituents selected from the group consisting of halogen atoms, hydroxyl groups, C₁-C₄ alkoxy groups, or C₆-C₁₂ aryl groups (which can be substituted by one to three halogen atoms). If the term alkyl alone or in combination with other terms is used without giving any hint to the length of the chain the chain is meant to have at least one to five carbon atoms and the chain may be straight or branched and can optionally be substituted by hydroxy, halogen, methoxy.

The term "aryl" as used herein refer to a monocyclic aromatic carbocyclic e.g., phenyl, or heterocyclic ring moiety e.g. furyl, thienyl, pyridyl, pyrazolyl, pyrimidinyl, oxazolyl, pyridazinyl, pyrazinyl, thiazolyl, containing five to 14 ring atoms. Aryl groups can be substituted by one or more substituents selected from the group consisting of halogen, hydroxy, (C₁-C₁₀)alkoxy, -CO₂H, -CO₂(C₁-C₁₀)alkyl, -NH₂, -NO₂, -N₃, -CN, (C₁-C₂₀)alkyl, (C₁-C₂₀)acyl, or (C₁-C₂₀)acyloxy. The heteroatoms can be oxidized, if as a result the aromatic character is not lost, e. g., a pyridine moiety can be oxidized to a pyridine N-oxide.or the term "aryl" refers to a bi- and tricyclic aryl radical, which can be a substituted or unsubstituted carbocyclic or heterocyclic radical with one or more heteroatoms, such as naphthyl, anthryl, benzoxazolyl, benzothiazolyl, benzimidazolyl, quinolyl, isoquinolyl, benzoxazinyl, benzofuranyl, indolyl, indazolyl, quinoxalinyl, tetrahydroisoquinolinyl, tetrahydroquinolinyl, thienopyridinyl, pyridopyridinyl, benzopyrazolyl, benzotriazolyl, or dihydroindolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a quinolyl to a quinolyl-N-oxide.

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy, -CO₂H, -CO₂Alkyl, -NH₂, -NO₂, -N₃, -CN, C₁-C₂₀ alkyl, C₁-C₂₀ acyl, or C₁-C₂₀ acyloxy.

The term "alkenyl" as used herein refers to straight or branched alkenyl groups, e.g. Vinyl-, Allyl-, Homoallyl-, (*E*)-But-2-enyl-, (*Z*)-But-2-enyl-, (*E*)-But-1-enyl-, (*Z*)-But-l-enyl-, Pent-4-enyl-, (*E*)-Pent-3-enyl-, (*Z*)-Pent-3-enyl-, (*E*)-Pent-2-enyl-, (*Z*)-Pent-2-enyl-, (*E*)-Pent-1-enyl-, (*Z*)-Pent-1-enyl-, 2-Methylvinyl-, 3-Methylbut-3-enyl-, 2-Methylbut-3-enyl-, (*E*)-2-Methylbut-2-enyl-, (*Z*)-2-Methylbut-2-enyl-, 3-Methylbut-2-enyl-Gruppe.

The term "alkynyl" as used herein refers to straight or branched alkynyl groups, e.g. C≡C, -CH₂-C≡CH, -C≡C-CH₃, -CH(CH₃)-C≡CH, -C≡C-CH₂(CH₃), -C(CH₃)₂-C≡CH, -C≡C-CH(CH₃)₂-, -CH(CH₃)-C≡C-CH₃, -CH₂-C≡C-CH₂(CH₃).

The term "alkoxy" as used herein refers to C₁-C₁₀-alkoxy groups, preferably C₁-C₅-alkoxy groups, that can be straight-chain or branched e.g. methoxy, ethoxy, n-propoxy, iso-propoxy, n-butoxy, iso-butoxy, tert-butoxy or n-pentoxy, 2,2-dimethylpropoxy, 2-methylbutoxy or 3-methylbutoxy group. A methoxy or ethoxy group is preferred.

The term "acyl" means -C(O)alkyl or -C(O)phenyl or -C(O)benzyl, the alkyl chain may be straight or branched and has up to 12 carbon atoms and the phenyl ring may optionally be substituted by halogen, hydroxyl, C₁-C₅-alkoxy, and the acyl residue is derived from the known carbonic acids such as, e.g. CH(O)OH, CH₃-COOH, CH₃-CH₂-COOH, CH₃-CH₂-CH₂-COOH, CH₃-CH₂-CH₂-CH₂-COOH, pivalic acid.

The term "hal" or "halogen" means fluorine, chlorine, bromine or iodine, preferably in end products fluorine or chlorine, as a leaving group preferably iodine or bromine.

The residue CH₂N(alkyl, acyl)_{1,2} means that the nitrogen can bear one or two same or different residues selected from alkyl or acyl and both terms, alkyl and acyl have the meaning as defined above.

The protecting groups PG^{z} can be alkyl- and/or aryl-substituted silyl moieties, such as tris(C₁-C₂₀ alkyl)silyl, bis(C₁-C₂₀ alkyl)-arylsilyl, (C₁-C₂₀ alkyl)-diarylsilyl, tris(aralkyl)-silyl, C₁-C₂₀ alkyl, C₂-C₂₀ alkenyl, C₄-C₇ cycloalkyl, which may contain an oxygen atom in the ring, aryl, aralkyl, C₁-C₂₀ acyl, aroyl, C₁-C₂₀ alkoxycarbonyl C₁-C₂₀ alkylsulfonyl or C₁-C₂₀ arylsulfonyl. Protecting groups which can be easily be removed from the molecule are preferred, e.g., methoxymethyl, methoxyethyl, ethoxyethyl, tetrahydropyranyl, tetrahydrofuranyl, trimethylsilyl, triethylsilyl, triisopropylsilyl, *t*-butyldimethylsilyl, *t*-butyl-diphenylsilyl, triphenylsilyl, tribenzylsilyl, benzyl, *p*-nitrobenzyl, *p*-methoxybenzyl, as well as alkylsulfonyl e.g. methylsulfonyl or arylsulfonyl e.g. p-tolylsulfonyl. As an alkoxycarbonyl radical, e.g., trichloroethyloxycarbonyl (Troc) is suitable. Preferred acyl groups are formyl, acetyl, propionyl, isopropionyl, trichloromethylcarbonyl pivaloyl, butyryl, or benzoyl, which all can be substituted by one or more amino and/or hydroxy moieties.

The etherified or esterified hydroxyl groups result e.g. in (C₁-C₅)-alkoxy or (C₁-C₅)-alkoxy(C₁-C₅)alkyl groups e.g. MEM (Methylethoxymethyl), MOM (Methyloxymethyl), or cyclic ethers as e.g.THP (Tetrahydropyranylether), O(CO)(C₁-C₁₀)alkyl groups, O(CO)benzyl, O(CO)phenyl, with the used term alkyl as defined above.

As amino protective groups PG, the radicals that are known to one skilled in the art are considered. For example, the Alloc, Boc, Z, benzyl, f-Moc, Troc, Stabase or benzostabase group can be mentioned.

In one aspect of the invention all compounds defined in formula I as well as their salts, solvates and solvates of salts are encompassed, especially the salts, solvates and salts of solvates of the compounds disclosed in the examples are one aspect of the invention as long as the disclosed compounds themselves are not already salts, solvates or solvates of the salts.

The compounds encapsulated in the nanoparticles of the present invention can exist in stereoisomeric forms such as enantiomers of diastereoisomers depending on their structure and residues as defined in formula I. In one aspect of the invention therefore all these enantiomers, diastereoisomers or mixtures thereof are encompassed. The isolation of enantiomerically or diastereomerically pure isomers if possible by methods of the state of the art, e.g. using column chromatography with a chiral solid phase.

Should it be possible that the compounds of the invention also exist in tautomeric forms these are also an aspect of the present invention.

The term physiologically unobjectable salts includes addition salts of mineral acids, carbonic acids, sulfonic acids, e.g. salts of hydrochloric acid, hydrobromic acid, sulfuric acid, nitric acid, phosphoric acid, methanesulfonic acid, ethanesulfonic acid, toluolsulfonic acid, benzenesulfonic acid, naphthalinesulfonic acid, acetic acid, trifluoroacetic acid, propionic acid, lactic acid, tartaric acid, malic acid, citric acid, fumaric acid, pivalic acid, maleic acid, succinic acid and benzoic acid.

In addition the term physiologically unobjectable salts includes salts of commonly suitable bases, e.g. salts of alkalimetall (e.g.. sodium- and potassium salts), alkaline earth salts (e.g. calcium- and magnesium salts) and ammonium salts, derivatized from NH₃ or organic amines with 1 to 16 carbon atoms, e.g. ethylamine, diethylamine, triethylamine, ethyldiisopropylamine, monoethanolamine, diethanolamine, triethanolamine, dicyclohexylamine, dimethylaminoethanol, prokaine, dibenzylamine, N-methylmorpholin, arginin, lysin, ethylendiamine and N-methylpiperidin.

The term "optionally substituted, aromatic or non-aromatic, 5- or 6-membered heterocyclic ring" preferably means a substituted or unsubstituted oxazolyl, thiazolyl or pyridyl. The substituents of the "optionally substituted, aromatic or non-aromatic, 5- or 6-membered heterocyclic ring" may be selected from the group consisting of C₁₋₄ alkyl, hydroxy C₁₋₄ alkyl, amino C₁₋₄ alkyl, C₁₋₄ alkyloxy and C₁₋₄ thioalkyloxy; preferably from the group consisting of methyl, hydroxymethyl, aminomethyl and thiomethoxy. Most preferably, the term "optionally substituted, aromatic or non-aromatic, 5- or 6-membered heterocyclic ring" means 2-methyl-thiazol-5-yl, 2-hydroxymethyl-thiazol-5-yl, 2-aminomethyl-thiazol-5-yl or 2-thiomethoxy-thiazol-5-yl.

The term "optionally substituted, aromatic or non-aromatic, 8- to 10-membered heterocyclic ring" means a substituted or unsubstituted benzoxazolyl or benzothiazolyl or quinolyl ring, preferably means a substituted or unsubstituted benzothiazolyl ring. The substituents of the "optionally substituted, aromatic or non-aromatic, 8- to 10-membered heterocyclic ring" may be selected from the group consisting of C₁₋₄ alkyl, hydroxy C₁₋₄ alkyl, amino C₁₋₄ alkyl, C₁₋₄ alkyloxy and C₁₋₄ thioalkyloxy; preferably from the group consisting of methyl, hydroxymethyl, aminomethyl and thiomethoxy. Most preferably, the term "optionally substituted, aromatic or non-aromatic, 8- to 10-membered heterocyclic ring" means 2-methyl-benzothiazol-5-yl.

Preferred group of drugs suitable as active drug substance for the preparation of the nanoparicles of the invention the epothilones or epothilone analogues as defined herein.
Further epothilones suitable for the invention are epothilone A, epothilone B (Epo-906), epothilone C, epothilone D (Kos-862), epothilone E, epothilone F, (E)-9,10-dehydro epothilone B (Kos-1584), 15-aza-epothilone B (BMS-247550), BMS-310705. ABJ 879, fludelone, or (1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

The especially preferred drug in the sense of the invention is
(1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicycto[14.1.0]heptadecane-5,9-dione.

It is commonly known in the art how to synthesize epothilones.
The epothilones as disclosed in the art e.g. WO 93/10102, WO 2000/066589, WO 2001027308 , WO 99/02514 , WO 2002080846are suitable for use in the present invention.

The epothilones and their derivatives as being suitable for the present invention as well as the production thereof is disclosed, for example in DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly interesting epothilones of the present invention may be produced according to WO 00/66589.
Some further information is provided in references 1-7.

Solvates in the sense of the invention are such forms of the compounds of the present combinations which build complexes by coordination of solvent molecules in a liquid or a solid phase. Hydrates are special forms of a solvate wherein water molecules are coordinated.

In the present context, the term "positively charged block-copolymer" refers to a block-copolymer wherein at least one moiety or functional element has a positive charge. The block-copolymer is preferably made up of a substantially equal number of copolymerized blocks, but the total number of blocks may be either even or odd.

In the present context the term "charge" reflects the physicochemical property of a chemical moiety in aqueous media at neutral pH, meaning a range from pH = 6,5-7,5.

In the present context, the term "biodegradable polymer" refers to a polymeric material that will be metabolized or hydrolytically or oxidatively degraded in the body upon administration.

In the context of the present invention, the term "polymer derived from a natural source" refers to a non-synthetic or semi-synthetic polymer having an original natural source. Preferred examples are negatively charged polymers as e.g. Alginic Acid, Hyaluronic acid, acidic cellulose derivatives, acidic starch derivatives, acidic polysaccharides, acidic polyamino acids, and acidic proteins.

In the context of the present invention the term "aqueous salt containing solution", optionally being used for diluting the block co-polymer, is a solution as recommended from the vendor of the block co-polymer for dilution and/or recommended for injection into warm blood animal.

In the context of the non-modified nanoparticles disclosed herein, the term "negative surface charge" is meant to describe nanoparticles with a zeta-potential of less than 0 mV. In order to determine the surface charge of the non-modified nanoparticles incorporated in the surface-modified nanoparticles of the invention, the corresponding non-modified nanoparticle is produced and the zeta-potential is measured. The zeta-potential of a nanoparticle is measured by the method of analyzing a 0.1 % [w/w] dispersion in a standard 4 side open cuvette using a distilled water as the dispersion medium and a Malvern Zetamaster equipment in standard mode. This procedure is used for all measurements of zeta-potenial where necessary.

The term "surface modified" is used in the context of the invention for nanoparticles having a non-modified core particle consisting of a biodegradable polymer and a drug and having a second layer made of a positivle charged copolymer leading to a surface modified particle having a zeta potential > 0 mV. This is achieved by titration methods which are carried out by stepwise addition of positively charged block co-polymer in solution and optionally subsequent or continous determination of the zeta potential.

In the context of the surface-modified nanoparticles of the invention, the term "positive surface charge" is meant to describe surface-modified nanoparticles with a zeta-potential greater than 0 mV or equal thereto.

In the present context when the term "particle size" is used it relates to particle sizes determined with the procedure described below:
The size of the non-modified and the surface modified particles was measured using dynamic light scattering. A "zetasizer 3000" of Malvern Instruments was used with standard equipment and software. 2 ml of the 0.1 % [w/v] dispersion of the particles in distilled water at neutral pH (pH 7.0) at room temperature in a cuvette were put into the laser beam at standard wavelength. The scattered light was measured at an angle of 90°. Volume weighted distribution was determined.

The term "cryoprotectants" as used herein generally include agents, which provide stability to the Epothilone from freezing-induced stresses. Examples of cryoprotectants include sugars, cyclodextrins such as β-cyctodextrins and derivatives thereof, and include polyols such as, for example, trehalose, mannitol, sorbitol, mannit, sorbit, higher molecular weight polymers like e.g. polyethylenglycol, cellulose derivatives like e.g. hydroxypropylcellulose, hydroxypropylmethylcellulose, saccharose, glucose as well as pH regulators. PEG derivatives such as PEG 4000 are preferred.,.
The pH regulators include amines, such as trometamol, mineral acids, organic acids, such as hydrochloric acid. Additionally surfactants can be used as cryoprotectants. Examples of surfactants include amines such as, trometamol. Cryoprotectants also contribute to the tonicity of the formulations. Cryoprotectants may also have lyoprotectant effects.

The term "lyoprotectant" as used herein includes agents that provide stability to the Epothilone during water removal in the drying process, such as during lyophilisation process. Examples of lyoprotectants include saccharides, in particular sugar alcohols in particular mannitol. Saccharides of interest are di- and tri-saccharides such as sucrose, dextrose, lactose, maltose and/or trehalose.

In the context of the drugload of the compositions of the present invention, the term "matrix polymer weight" refers to the weight percentage of the active drug substance relative to the weight of the biodegradable polymer used to form the drug loaded nanoparticles plus weigth of the active drug.

In the context of the present invention the term "water" if not defined further is mainly to be water for injection or infusion.

The term "organic solvent" in the sense of the invention for dissolution of the drug is any solvent suitable to completely dissolve the compound. In the event of epothilones or its derivatives a polar aprotic solvent e.g. alcohols such as methanol, ethanol, propanol, isopropanol; tetrahydrofurane; dimethylsulfoxide; methylacetate, preferably acetone, are suitable.

As solvent to solve any biodegradable polymer or mixtures thereof the following solvents may be selected of the list consisting of e.g. alcohols such as methanol, ethanol, propanol, isopropanol; tetrahydrofurane; dimethylsulfoxide; methylacetate, preferably acetone.

For preparation of a solution of a positively charged block-copolymer the following solvents are preferably suitable: water, alcohols like methanol, ethanol, propanol, isopropanol and mixtures thereof.

The term "non-solvent" of a drug is meant to be a solvent which does not solve the drug, for the purposes of epothilones especially water is suitable as well as aqueous solutions of salts e.g. sodiumchloride (saline), mixtures of low molecular polyethylenglycol/water.

The term "lyophilizing" means the method for removing a solvent under low pressure and low temperatures. The method used is described in the experimental section.

### Detailed description

The pharmaceutical composition according to the present invention comprising surface-modified nanoparticles, wherein said nanoparticles contain an active drug substance, and wherein one or more positively charged block-copolymers are bound to the surface of said nanoparticles, may in principle contain any active drug substance. However, in a preferred embodiment, the active drug substance comprised in the compositions according to the present invention is an anti-tumor drug such as epothilones or epothilone analogues, such as epothilone A, epothilone B (= Epo-906 = Patupilone), epothilone C, epothilone D (= Kos-862), epothilone E, epothilone F, (E)-9,10-dehydro epothilone B (= KOS-1584), KOS-1803 or 15-aza-epothilone B (= BMS-247550 = Ixabepilone), BMS-310705, ABJ 879, Fludelone.

### Non-modified nanoparticles

Many different nanoparticles are known in the art (see reference 8). The composition of the nanoparticle preferably comprises a polymer, such as a biodegradable polymer or a mixture of biodegradable polymers. The biodegradable polymer may be defined in terms of the monomers that are polymerized to obtain the polymer, or it may be defined by its polymer name.

In one embodiment, said biodegradable polymer is a polymer derived from a natural source or a homo- or co-polymer of a monomer selected from the group consisting of lactide, such as I-lactide or dl-lactide, glycolide, dioxanone, trimethylene carbonate, alkylcyanoacrylate, such as butylcyanoacrylate or isobutylcyanoacrylate, sialic acid, caprolactones, such as ε-caprolactone, β-malic acid, glycerol-adipate, sebacic acid, nucleotides as well as any mixtures thereof.

In another embodiment, said biodegradable polymer is selected from the group consisting of polylactide, such as poly(dl-lactide) or poly(I-lactide), polyglycolide, polydioxanone, poly(glycolide-co-trimethylene carbonate), polylactide-co-glycolide (PLGA), such as poly(I-lactide-co-glycolide) or poly(dl-lactide-co-glycolide), poly(I-lactide-co-dl-lactide), poly(glycolide-co-trimethylene carbonate-co-dioxanone), and polyalkylcyanoacrylate, such as polybutylcyanoacrylate or polyisobutylcyanoacrylate, alginic acid, hyaluronic acid, polysialic acid, acidic cellulose derivatives, acidic starch derivatives, acidic poly saccharides, acidic polyamino acids, acidic proteins, poly(β-hydroxybutyrate), polysebacic acid polyanhydrides, polyorthoesters, polycaprolactones, such as poly-ε- or γ-caprolactone, as well as any mixture and copolymers thereof. The biodegradable polymers used for the present invention are all bought from external sources and not synthesized.

In a preferred embodiment, said biodegradable polymer is a polyalkylcyanoacrylate, such as polybutylcyanoacrylate (PBCA) or polyisobutylcyanoacrylate, most preferred polybutylcyanoacrylate (PBCA).

11 In another preferred embodiment, said biodegradable polymer is a polylactide-co-glycolide (PLGA), including all the marketed Resomers of Boehringer Ingelheim.

The degree of polymerisation may vary and may result in a higher or lower molecular weight of the polymer. Accordingly, the molecular weight of the biodegradable polymer may be in the range of 10.000 - 10.000.000 Da, such as in the range of 15.000 - 5.000.000 Da, e.g. in the range of 20.000 - 2.000.000 Da, 15.000 - 2.000.000 Da, preferably in the range of 15.000 - 50.000 Da, as indicated by the supplier.

The surface-modified nanoparticles according to the present invention have many advantages. One particular advantage is the ability of negatively charged non-modified nanoparticles to bind positively charged block-copolymers by means of electrostatic forces. Hence, in a preferred embodiment of the present invention, the non-modified nanoparticles have a negative surface charge. The non-modified nanoparticles with a negative charge may be defined as having a zeta-potential in the range < 0 mV to -150 mV, such as in the range < 0 mV to -100 mV, preferably in the range < 0 mV to -50 mV, such as in the range -10 mV to -40 mV, when measured by the method described herein.

For obtaining the surface modified nanoparticles a second polymer is added to the so obtained non-modified particles. Polymers especially suitable for the surface modification are block-copolymers. The positively charged block-copolymer is bound to the surfaces of said non-modified core nanoparticle mainly by means of electrostatic forces (claim 13).
Thus one embodiment of the invention is a composition comprising surface modified nanoparticles wherein the core of said nanoparticle is a non-modified nanoparticle comprising a drug and the surface modification is achieved by binding of a block-copolymer to its surface.

### Block-copolymers

The positively charged block-copolymers comprised in the compositions according to the present invention preferably have the general formula (I)

(Y-)ₒ(X-Y)ₙ(-X)ₘ , (I)

wherein X is a non-charged polymer, Y is a positively charged polymer, o is 0 or 1, m is 0 or 1, and n is an integer from 1 to 10, such as 1 to 5, e.g. 1 to 3. The integer n may thus be 1, 2, 3, 4, 5, 6, 7, 8, 9 or 10. Preferably, n is 1.

The non-charged polymer X is consisting of a number of monomer units in the range as indicated below.

The non-charged polymer, X, may be selected from the group consisting of water
soluble starch and cellulose derivatives, such as
   maize starch, corn starch, potato starch, chemically modified starch, Hydroxy Ethyl Methyl Cellulose (HEMC), Hydroxy Propyl Methyl Cellulose (HPMC), Methyl Cellulose (MC), Hydroxy Ethyl Cellulose (HEC), Methyl Hydroxyethyl Cellulose (MHEC), Methyl hydroxypropyl Cellulose (MHPC), Tylose, or Methocel,
   number of monomers in the sense of the invention 100 - 2000, preferred 250 - 750, more preferred 500-2000(?)
water soluble polyacrylic acid derivatives, such as
   polymethacrylic acid, poly(methyl methacrylic acid),
   poly(ethyl methacrylic acid), poly(propyl methacrylic acid), poly acrylic acid, poly(methyl acrylic acid), poly(ethyl acrylic acid), poly(propyl acrylic acid), number of monomers in the sense of the invention 50 - 1000, preferred 100 - 500
and polyethyleneglycol (PEG),
   number of monomers in the sense of the invention 50 - 500, preferred 100 - 200
as well as any mixture and co-polymer thereof.

In a preferred embodiment X is polyethyleneglycol (PEG).

The positively charged polymer, Y, may be selected from the group consisting of polyethylenimine; polyalkylamine derivatives, such as
Polyalkylamin HCl; Chitosan; basic or in water positively charged polyaminoacids or proteins, such as homo- or co-polymers of Lysin, Arginin or Histidin, e.g. Polylysin, Polyarginin or Polyhistidin;
number of monomers in the sense of the invention 5 - 500, preferred 10 - 100,
basic Polyacrylic acid derivatives, such as
poly amino methacrylic acid, poly amino acrylic acid; and
poly-diallyldimethylammonium chloride (poly-DADMAC),
number of monomers in the sense of the invention 5 - 500, preferred 10 - 100,
as well as any mixture, copolymer or derivative thereof.

In a preferred embodiment, Y is poly-diallyldimethylammonium chloride (poly-DADMAC), poly-ethyleneimine, or poly-aminomethacrylic acid, in particular poly-DADMAC.

In another preferred embodiment, the positively charged block-copolymer is a block-copolymer of PEG and poly-DADMAC, especially PEG-polyDADMAC-PEG, a block-copolymer of PEG and poly-ethyleneimine or a block-copolymer of PEG and poly-aminomethacrylic acid.

The molecular weight of the positively charged block-copolymer will depend on the degree of polymerisation of each block and on the number of blocks. In one embodiment, the molecular weight of the positively charged block-copolymer is in the range 200 - 250.000 Da, such as 1000 - 200.000 Da, e.g. 2000 - 100.000 Da, preferably 5000 - 75.000 Da.

Block-copolymers may be manufactured in the manner known to the person skilled in the art. They may in particular be manufactured as devised by Lieske and Jaeger [9].

### Charge of the surface-modified nanoparticles

In a preferred embodiment, the amplitude of the total charge of the positively charged block-copolymers will at least equal the amplitude of the total negative charge of the non-modified nanoparticles. In certain cases, the number of positively charged block-copolymers bound to the surface of the non-modified nanoparticles is such that there are positive charges in the block-copolymer that are unmatched by negative charges in the non-modified nanoparticles. In such an embodiment, the surface-modified nanoparticles have a positive surface charge.

Said positive surface charge may be defined in terms of the zeta-potential which is the measurable surface charge of a shear plane. Therefore if the zeta potential is zero that does not necessarily mean that the surface charge is zero as well. In one embodiment, the zeta-potential of the surface-modified nanoparticles is in the range 0 mV to 150 mV, such as in the range 0 mV to 100 mV, preferably in the range 0 mV to 50 mV, such as in the range 10 mV to 40 mV, when measured by the method described herein.

### Size of the surface-modified nanoparticles

In one embodiment, the surface-modified nanoparticles have an average particle diameter of less than 1000 nm, such as less than 750 nm, e.g. less than 500 nm. In a preferred embodiment, the surface-modified nanoparticles have an average particle diameter in the range from 2-500 nm, such as in the range from 2-400 nm, e.g. in the range from 2-300 nm, preferably in the range from 2-250 nm, such as in the range from 2-200 nm, e.g. in the range from 5-200 nm. The method for measurement of the particle size is described in the definitions section.

### Administration

Various drug loads may be contained in the compositions according to the present invention, depending on the chosen active drug substance, the disease to be cured, the patient to be treated etc. In one embodiment, the drug load may be in the range 0.01 - 50% w/w of the matrix polymer weight, such as 0.02 - 25% w/w, e.g. 0.05 - 5% w/w.

Various administration forms for the compositions according to the present invention may be chosen, such as suspension, dispersion, gels, ointments, crèmes, sprays, mists, solid dispersions, powders. In a preferred embodiment, the compositions according to the invention are in the form of a suspension or other kinds of dispersions in a liquid. Also, in one embodiment, the compositions according to the invention are comprised in a unit dosage form.

### Pharmaceutical kits

The compositions according to the present invention may be prepared and stored ready for administration. As an alternative, the components of the composition may be kept separately and mixed shortly prior to administration. A second aspect of the invention therefore relates to a pharmaceutical kit comprising:
i) A first composition comprising nanoparticles, wherein said nanoparticles contain an active drug substance; and
ii) a second composition comprising a positively charged block-copolymer.

In said pharmaceutical kit, the active drug substance in i) may be ,any epothilone or epothilone analogue as defined above. Also, in one embodiment, the composition in i) may be lyophilised. In another embodiment, the composition in ii) may be an aqueous solution. In a particular embodiment, the composition in i) is lyophilised and the composition in ii) is an aqueous solution.

The nanoparticles comprised in said first composition in i) may be as defined in any of the embodiments above. Similarly, the positively charged block-copolymers comprised in said second composition in ii) may be as defined in any of the embodiments above.

In one embodiment, the pharmaceutical kit comprises:
i) a first composition comprising nanoparticles made from polylactide-co-glycolide (PLGA) containing an epothilone or epothilone analogue; and
ii) a second composition comprising a block-copolymer of PEG and poly-DADMAC.

In another embodiment the pharmaceutical kit comprises a composition containing the surface-modified nanoparticles, especially the surface modified nanoparticles obtained by combination of the solutions (i) and (ii) mentioned above.

In yet another embodiment the pharmaceutical kit comprises the lyophilisate of surface modified nanoparticles optionally together with means for reconstitution.

Means for reconstitution may be e.g. water for injection, physiological saline solution.

### Medical use

It will be evident to the person skilled in the art that the compositions and pharmaceutical kits according to the present invention may be used in medical treatment, in particular in the treatment of cancer.

One aspect of the invention therefore relates to any composition as defined above for use as a medicament. In another aspect, the invention relates to any composition or pharmaceutical kit according to the present invention for the manufacture of a medicament for the treatment of cancer, in particular to intravenous administration of said medicament.

In yet another aspect, the present invention relates to a method for the treatment of cancer, said method comprising administering a composition as defined in any of the embodiments above to a patient in need thereof, in particular to intravenous administration.

### Preparation of the compositions

It will be evident to the person skilled in the art how to prepare the compositions of the invention. One method for preparing the compositions comprises the steps:
a) Dissolving a biodegradable polymer or a mixture of biodegradable polymers in an organic solvent;
b) Combining solutions a) and b);
c) Diluting c) with a non-solvent of the drug and the biodegradable polymer(s) to form nanoparticles
d) Optionally lyophilizing;
e) Optionally diluting with water;
f) Optionally removing the solvent using e.g. temperature methods (evaporation) or filtration methods (e.g. microdialysis)
g) Preparing a solution of a positively charged block-copolymer; and Combining the solutions of d), e), f) or g) with the solution of h).

The solution of positively charged block-copolymer may be prepared by weighting of the appropriate amount of block-copolymer, adding water for injection, which may be buffered or adjusted to a suitable osmotic pressure to be used as an injection medium, and stirring till a clear solution is reached.

The following examples are intended to illustrate the invention but should not be read as a limitation thereof.

### Examples

### Preparation of a surface-modified nanoparticle according to the invention General procedure

Compounds used:
Epothilone:
(1S,3S,7S, 10R,11S,12S,16R)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione

### Non-modified Polymer:

RG-752 S, PLGA of Boehringer Ingelheim, Material number: 64362; the molecular weight as due to the vendors document is about 20.000 g/mol

PEG-DA: Polyethylenglycol-poly-diallyldimethylammonium chloride - Polyethylenglycol = PEG-DADMAC-PEG block co-polymer, whereby the ratio of the three building blocks is:1:2,8:1 (PEGDA 2000), blocks (+) DADMAC = 123, Molecular weight = 17.000 g/mol

### Procedure:

- Dissolve 1.5 mg of epothilone in 150 µl of acetone
- Dissolve 50 mg of RG-752 S in 2 ml of acetone
- Combine the acetonic solutions of the RG polymer and the epothilone
- Transfer the 2,15 ml solution into a syringe; adapt a 0.9 mm diameter canule onto the syringe
- Inject the acetonic solution within 2 seconds into a volume of 6 ml of water (optionally buffered, optionally osmotically adjusted) in a 20 ml beaker to form the nanoparticles by precipitation
- Stir it at 100 rpm for 24 h in the uncovered beaker to remove solvent (optionally vacumm and heat or lyophilisation)
- Dissolve PEGDA, which is in water (optionally buffered, optionally osmotically adjusted) to form a 0.01 % [w/v] solution
- Add 1.4 of the PEGDA solution to the nanoparticles dispersion
- Stir it for 2 minutes at 100 rpm
- Transfer it to a syringe; adapt canule suitable for injection (optionally adapt 0.2 µm filter)
- Injection into warm blooded animal incl. human

### Lyophilisation

The lyophilisation was startet after filling 3 ml of the dispersion and 300 mg PEG 4000 in 10 ml glas vials. The vials were frozen at minus 28 °C for 2 hours. Then they were placed into a "AdVantage AL" freeze dryer and lyophilised using following (Rezept 0002) program:
The terms R and H refer to machine terms, can be found in the table below and have the meaning:
R = Ramp (modification)
H = Hold (no modification)

| **Rezept 0002** | | | | |
|---|---|---|---|---|
| | | | | |

| 1.) Thermal Treatment Menü | | | | |
|---|---|---|---|---|
| | | | | |

| Step # | Temp. (°C) | Time (min) | R/H | |
|---|---|---|---|---|
| 1 | -035 | 0035 | R | |
| 2 | -035 | 0120 | H | |
| 3 | 000 | 0000 | H | |
| 4 | 000 | 0000 | H | |
| 5 | 000 | 0000 | H | |
| 6 | 000 | 0000 | H | |
| 7 | 000 | 0000 | H | |
| 8 | 000 | 0000 | H | |
| 9 | 000 | 0000 | H | |
| 10 | 000 | 0000 | H | |
| 11 | 000 | 0000 | H | |
| 12 | 000 | 0000 | H | |
| | | | | |

| 2.) FCV Menü | | | | |
|---|---|---|---|---|
| | | | | |
| Freeze Temperatur (°C) | | | -035 | |
| Additional Freezetime (min) | | | 0000 | |
| Condenser Setpoint (°C) | | | -060 | |
| Vaccum Setpoint (mTorr) | | | 0800 | |
| | | | | |

| 3.) Drying Phase Menü | | | | |
|---|---|---|---|---|
| | | | | |

| Step # | Temp. (°C) | Time (in) | accum (mTorr | R /H |
|---|---|---|---|---|
| 1 | -035 | 0030 | 0146 | H |
| 2 | -030 | 0030 | 0146 | R |
| 3 | -030 | 0720 | 0146 | H |
| 4 | -010 | 0030 | 0146 | R |
| 5 | -010 | 0360 | 0146 | H |
| 6 | 005 | 0060 | 0010 | R |
| 7 | 005 | 0240 | 0010 | H |
| 8 | 000 | 0000 | 0000 | R |
| 9 | 000 | 0000 | 0000 | H |
| 10 | 000 | 0000 | 0000 | H |
| 11 | 000 | 0000 | 0000 | H |
| 12 | 000 | 0000 | 0000 | H |
| 13 | 000 | 0000 | 0000 | H |
| 14 | 000 | 0000 | 0000 | H |
| 15 | 000 | 0000 | 0000 | H |
| 16 | 000 | 0000 | 0000 | H |
| 2nd (OFF) | 035 | | | |
| Post Heat | 005 | 0600 | 0100 | H |

### Example 1

The epothilone loaded nanoparticles were manufactured by, in a first step, combining a solution of 1.5 mg of the drug in 150 µl of acetone (HPLC grade) and 50 mg of PLGA (RG 752 Resomer by Boehringer Ingelheim) dissolved in 2 ml of Acetone. The 2.15 ml of acetonic solution were transferred into a syringe, having a max. filling volume of 5 ml, than a 0.9 mm needle was attached to the syringe. Within 2 seconds the complete volume was injected into 6 ml of pure water provided in a 20 ml beaker. The dispersion immediately turned opaque, indicating the formation of nanoparticles. The dispersion was stirred at 100 rpm under room conditions for 24 h in the open beaker to remove the acetone. The absent of acetone was tested by sensual investigations. Water loss during the removal of acetone was adjusted to obtain 6 ml of water based dispersion.
In another beaker the PEG-DA polymer was dissolved in pure water to form a 0.01 % [w/v] solution. 1.4 ml of the PEG-DA solution was added to the 6 ml of nanoparticles dispersion and stirred for 2 minutes at 100 rpm and under room conditions. The particles of this dispersion had a diameter of 190 nm as measured using the standard size determination measurement. The zetapotential was determined to be at 10 mV.
The dispersion was transferred into a 10 ml syringe, a suitable needle was attached.

### Example 2

750 mg of PLGA RG752 S and 30.0 mg of the epothilone were dissolved in 60 ml of acetone. The solution was injected within 20 seconds in 90 ml of an aqueous 1% [w/v] Synperonic F68 (Serva) solution using a 0.9 mm needle attached to a 100 cartridge. The dispersion immediately turned opaque. The dispersion was transferred into a 200 ml beaker and stirred at 100 rpm for 24 h open to remove the acetone. The absent of acetone was tested by sensual investigations. Water loss during the removal of acetone was adjusted to obtain 60 ml of water based dispersion. The dispersion was filtrated using ultracentrifugation cells (company Amicon, cut-off 100,000 g/mol) at 1000 g in a lab centrifuge to remove non encapsulated epothilone. The particles of this dispersion had a diameter of 157 nm as measured using the standard size determination measurement. The zetapotential was determined to be at -30 mV.
24 ml of residue was put into 8 vials with each containing 3 ml. In each vial 300 mg of polyethylenglycol (PEG) with a molecular size of 4000 g/mol (as determined by the supplier Fluka) was added. The dispersion was lyophilized using the standard procedure.
After redispersion in water the particles had diameter of 157 nm and a zetapotential of - 30 mV. The content of epothilone was determined using standard HPLC methods with internal calibration tube at 0.9 mg. For animal trials the lyophilisation product was resdispersed in 1 ml isotonic NaCl solution for injection. Stepwise addition of a 0.1 % [w/v] solution of PEG-DA in water was monitored by zetapotential determinations. Addition was stopped at 10 mV.
The dispersion (1.05 ml) was transferred into a 2 ml syringe, a suitable needle was attached.

### Example 3

The dispersion of example 2 was injected into Mamma carcinoma (MaTu) carrying nude mice for organ distribution determination.
6 male nude mice of the species NMRI nu/nu were used. 0.21 ml of the dispersion was injected i.v. into 3 mice to achieve a dose of 8 mg/kg for each mouse. The other 3 mice got equivalent doses of epothilone using standard water based solutions for injection. At 1, 2 and 4 hours post i.v. injection blood samples were collected and the whole tumor was removed from a single mouse for each time point.
Blood samples and tumor were treated using standard operations for biological samples to determine the epothilone with LC/MS coupling and internal standards as well as external calibration.
The result was a ratio [epothilone in Tumor tissue [µM]/ epothilone in blood sample [µM]] of 16.3, 29.1 and 27.7 at 1, 2 and 4 hours in the control group (standard water based injection) compared to 25.6, 35.0 and 72.3 at 1, 2 and 4 hours in the group treated with dispersion and particles of example 2.

### Example 4

The dispersion of example 2 was injected into Mamma carcinoma (MaTu) carrying nude mice for tumor regression and body weight determination.
Male nude mice of the species NMRI nu/nu were used. 0.21 ml of the dispersion was injected i.v. into 3 mice to achieve a dose of 8 mg/kg for each mouse. Other male nude mice of above species got equivalent doses using standard water based injection medium. Other male nude mice of above species got the particles of this invention without encapsulated epothilon. Tumor area was determined using in-vivo size determination (palpation). Body weight was determined by weighing. Treatment with i.v. injection was started at day 4 after tumor transplantation. Results at day 18 after transplantation (day 14 after injection) were as follows:

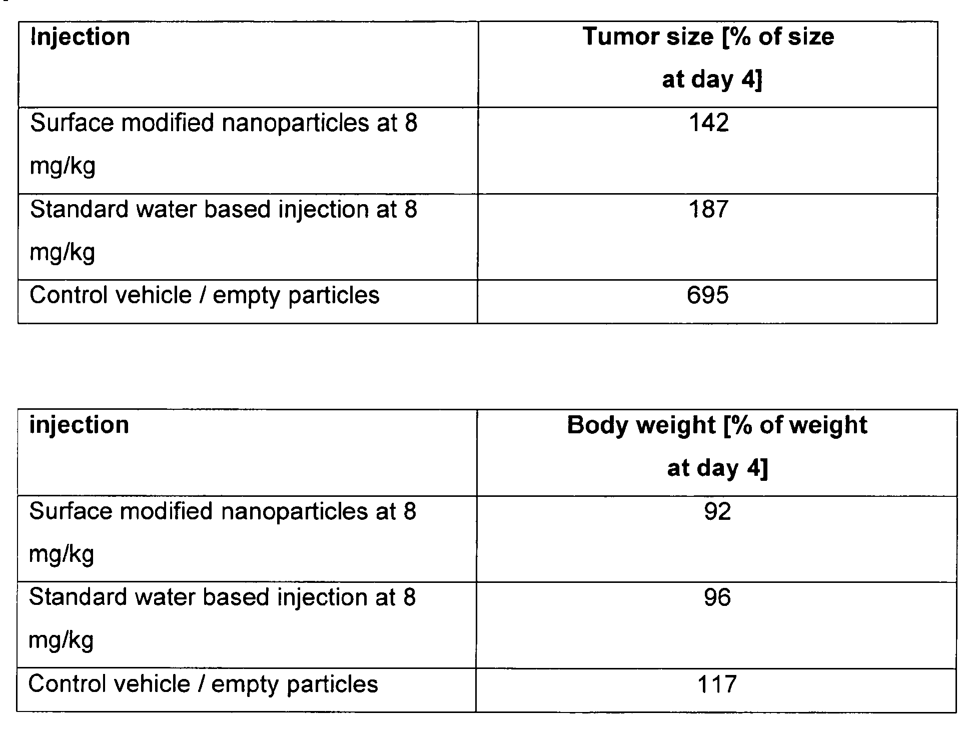

### References

1. Luduvico, I.; Hyaric, M. L.; Almeida, M. V.; Da Silva, A. D. Mini-Reviews in Organic Chemistry 2006, 3, 49-75. (Review)
2. Balog, D. M.; Meng, D.; Kamanecka, T.; Bertinato, P.; Su, D.-S.; Sorensen, E. J.; Danishefsky, S. J. Angew. Chem. 1996, 108, 2976.
3. Su, D.-S.; Meng, D.; Bertinato, P.; Balog, D. M.; Sorensen, E. J.; Danishefsky, S. J.; Zheng, Y.-H.; Chou, T.-C.; He, L.; Horwitz, S. B. Angew. Chem. Int. Ed. Engl. 1997, 36, 757.
4. Yang, Z.; He, Y.; Vourloumis, D.; Vallberg, H.; Nicolaou, K. C. Angew. Chem. Int Ed. Engl. 1997, 36, 166.
5. Schinzer, D.; Limberg, A.; Bauer, A.; Böhm, O. M.; Cordes, M. Angew. Chem. Int. Ed. Engl. 1997, 36, 523.
6. Mulzer, J.; Mantoulidis, A.; Öhler, E. J. Org. Chem. 2000, 65, 7456-7467.
7. Bode, J. W.; Carreira, E. M. J. Am. Chem. Soc. 2001, 123, 3611-3612.
8. Yezhelyev, M. V.; Gao, X.; Xing, Y.; Al-Hajj, A.; Nie, S.; O'Regan, R. M. The Lancet Oncology 2006, 7, 657-667.
9. Lieske, A.; Jaeger, W., Macromol. Chem. Phys. 1998, 199, 255-260.

## Claims

1. A pharmaceutical composition comprising surface-modified nanoparticles, wherein the core of said nanoparticles is a non-modified nanoparticle containing an active drug substance, and wherein one or more positively charged block-copolymers are bound to the surface of said nanoparticles.

2. The composition according to claim 1, wherein said nanoparticles comprise a biodegradable polymer or a mixture of biodegradable polymers.

3. The composition according to claim 2, wherein said biodegradable polymer is a polymer derived from a natural source or a homo- or co-polymer of a monomer selected from the group consisting of lactide, such as I-lactide or dl-lactide, glycolide, dioxanone, trimethylene carbonate, alkylcyanoacrylate, such as butylcyanoacrylate or isobutylcyanoacrylate, sialic acid, as well as any mixtures thereof.

4. The composition according to claim 3, wherein said biodegradable polymer is selected from the group consisting of polylactide, such as poly(dl-lactide) or poly(I-lactide), polyglycolide, polydioxanone, poly(glycolide-co-trimethylene carbonate), polylactide-co-glycolide (PLGA), such as poly(I-lactide-co-glycolide) or poly(dl-lactide-co-glycolide), poly(I-lactide-co-dl-lactide), poly(glycolide-co-trimethylene carbonate-co-dioxanone), and polyalkylcyanoacrylate, such as polybutylcyanoacrylate or polyisobutylcyanoacrylate, Alginic Acid, Hyaluronic acid, polysialic acid, acidic cellulose derivatives, acidic starch derivatives, acidic poly saccharides, acidic polyamino acids, acidic proteins, as well as any mixture thereof.

5. The composition according to claim 4, wherein said biodegradable polymer is a polyalkylcyanoacrylate.

6. The composition according to claim 5, wherein said polyalkylcyanoacrylate is polybutylcyanoacrylate (PBCA) or polyisobutylcyanoacrylate.

7. The composition according to claim 6, wherein said polyalkylcyanoacrylate is polybutylcyanoacrylate (PBCA).

8. The composition according to claim 4, wherein said biodegradable polymer is a polylactide-co-glycolide.

9. The composition according to any one of claims 2 to 8, wherein the molecular weight of the biodegradable polymer is 10.000 - 10.000.000 Da, 15.000 - 5.000.000 Da, or 15.000 - 2.000.000 Da.

10. The composition according to claim 1, wherein the non-modified nanoparticles of the core have a negative surface charge.

11. The composition according to claim 10, wherein the zeta-potential of said non-modified nanoparticles is in the range 0 mV to -150 mV, such as in the range 0 mV to -100 mV, preferably in the range 0 mV to -50 mV, such as in the range -10 mV to -40 mV, in water.

12. The composition according to claim 1, wherein the active drug substance is an epothilone or an epothilone analogue.

13. The composition according to claim 1, wherein said positively charged block-copolymer is bound to the surfaces of said non-modified core nanoparticle by means of mainly electrostatic forces.

14. The composition according to any one of the preceding claims, wherein said positively charged block-copolymer has the general formula (I)
(Y-)ₒ(X-Y)ₙ(-X)ₘ , (I)
wherein X is a non-charged polymer, Y is a positively charged polymer, o is 0 or 1, m is 0 or 1, and n is an integer from 1 to 10.

15. The composition according to claim 14, wherein X is selected from the group consisting of water soluble starch and cellulose derivatives, such as maize starch, corn starch, potatoe starch, chemically modified starch, Hydroxy Ethyl Methyl Cellulose (HEMC), Hydroxy Propyl Methyl Cellulose (HPMC), Methyl Cellulose (MC), Hydroxy Ethyl Cellulose (HEC), Methyl Hydroxyethyl Cellulose (MHEC), Methyl hydroxypropyl Cellulose (MHPC), Tylose, or Methocel, water soluble polyacrylic acid derivatives, such as polymethacrylic acid, poly(methyl methacrylic acid), poly(ethyl methacrylic acid), poly(propyl methacrylic acid), poly acrylic acid, poly(methyl acrylic acid), poly(ethyl acrylic acid), poly(propyl acrylic acid), and polyethyleneglycol (PEG), as well as any mixture and co-polymer thereof.

16. The composition according to claim 15, wherein X is PEG.

17. The composition according to any one of claims 14-16, wherein Y is selected from the group consisting of polyethylenimine; polyalkylamine derivatives, such as Polyalkylamin HCl; Chitosan; basic or in water positively charged polyaminoacids or proteins, such as homo- or co-polymers of Lysin, Arginin or Histidin, e.g. Polylysin, Polyarginin or Polyhistidin; basic Polyacrylic acid derivatives, such as poly amino methacrylic acid, poly amino acrylic acid; and poly-diallyldimethylammonium chloride (poly-DADMAC), or any mixture, copolymer or derivative thereof.

18. The composition according to claim 17, wherein Y is poly-DADMAC.

19. The composition according to any one of claims 14-18, wherein n is 1.

20. The composition according to any one of claim 14, wherein said positively charged block-copolymer is a block-copolymer of PEG and poly-DADMAC.

21. The composition according to any one of claims 14-20, wherein the molecular weight of said positively charged block-copolymer is in the range 200 - 250.000 Da, such as 1000 - 200.000 Da, preferably 2000 - 100.000 Da, more preferably 5000 - 75.000 Da.

22. The composition according to any one of the preceding claims, wherein said surface-modified nanoparticles have a positive surface charge.

23. The composition according to claim 22, wherein the zeta-potential of said surface-modified nanoparticles is in the range 0 mV to 150 mV, such as in the range 0 mV to 100 mV, preferably in the range 0 mV to 50 mV, such as in the range 10 mV to 40 mV, in water.

24. The composition according to any one of the preceding claims, wherein said surface-modified nanoparticles have an average particle diameter of less than 1000 nm, such as less than 750 nm, e.g. less than 500 nm.

25. The composition according to claim 24, wherein said surface-modified nanoparticles have an average particle diameter in the range from 2-500 nm, such as in the range from 2-400 nm, e.g. in the range from 2-300 nm, preferably in the range from 2-250 nm, such as in the range from 2-200 nm, e.g. in the range from 5-200 nm.

26. The composition according to any one of claims 12-25, wherein said epothilone is (1S,3S,7S,10R,11 S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

27. The composition according to any one of the preceding claims, wherein the drug load is in the range 0.01 - 50% w/w of the matrix polymer weight, such as 0.02 - 25% w/w, e.g. 0.05 - 5% w/w.

28. The composition according to any one of the preceding claims, wherein said composition is in the form of a suspension.

29. A unit dosage form comprising a composition as defined in any one of the preceding claims.

30. A pharmaceutical kit comprising:
i) A first composition comprising nanoparticles, wherein said nanoparticles contain an active drug substance; and
ii) a second composition comprising a positively charged block-copolymer.

31. The kit according to claim 30, wherein the active drug substance in i) is an epothilone or epothilone analogue.

32. The kit according to claim 30 or 31, wherein said first composition is lyophilised.

33. The kit according to any one of claims 30-32, wherein said second composition is an aqueous solution.

34. The kit according to any one of claims 30-33, wherein said nanoparticles are as defined in any one of claims 2-11.

35. The kit according to any one of claims 30-34, wherein said positively charged block-copolymer is as defined in any one of claims 14-21.

36. A composition according to any one of claims 1-29 for use as a medicament.

37. Use of a composition according to any one of claims 1-29 for the manufacture of a medicament for the treatment of cancer.

38. Use according to claim 37, wherein said medicament is administered intravenously.

39. A method for the treatment of cancer, said method comprising administering a composition as defined in any one of claims 1-29 to a patient in need thereof.

40. The method according to claim 39, wherein said composition is administered intravenously.

41. A method for the preparation of the composition according to any one of claims 1-29 comprising the steps
a) Dissolving a biodegradable polymer or a mixture of biodegradable polymers in an organic solvent;
b) Combining solutions a) and b);
c) Diluting c) with a non-solvent of the drug and the biodegradable polymer(s) to form nanoparticles
d) Optionally lyophilizing;
e) Optionally diluting with water;
f) Optionally removing the solvent using e.g. temperature methods (evaporation) or filtration methods (e.g. microdialysis)
g) Preparing a solution of a positively charged block-copolymer; and Combining the solutions of d), e), f) or g) with the solution of h).
